# EUROPÄISCHE PATENTSCHRIFT

(11) **EP 1 126 912 B1**
(45) Veröffentlichungstag und Bekanntmachung des Hinweises auf die Patenterteilung: **05.04.2006**
(21) Anmeldenummer: 00935201.4
(22) Anmeldetag: 10.06.2000
(51) Int. Cl.: B01J 19/00

(54) **KIT ZUR DURCHFÜHRUNG BIOCHEMISCHER REAKTIONEN MIT HOHEM DURCHSATZ**
KIT FOR CARRYING OUT BIOCHEMICAL REACTIONS WITH A HIGH THROUGHPUT
KIT DE REALISATION DE REACTIONS BIOCHIMIQUES A DEBIT ELEVE

(30) Priorität: 18.06.1999 EP 99111762
(43) Veröffentlichungstag der Anmeldung: 29.08.2001
(73) Patentinhaber: Roche Diagnostics GmbH, 68305 Mannheim (DE)
(72) Erfinder: METZLER, Thomas, D-80337 München (DE); SCHELS, Hans, D-80687 München (DE); REICHHUBER, Rolf, D-82377 Penzberg (DE); KLUGE, Jochen, D-81476 München (DE)
(86) Internationale Anmeldenummer: PCT/EP2000/005378
(87) Internationale Veröffentlichungsnummer: WO 2000/078444

(56) Entgegenhaltungen:
- EP-A- 0 894 852
- WO-A-97/04074
- WO-A-99/50436
- DE-A- 3 723 004
- DE-A- 4 237 113
- US-A- 4 642 220
- US-A- 5 362 624
- US-A- 5 478 730
- US-A- 5 593 856
- KIM D M ET AL: "A semicontinuous prokaryotic coupled transcription/translation system using a dialysis membrane." BIOTECHNOLOGY PROGRESS., Bd. 12, Nr. 5, September 1996 (1996-09), Seiten 645-649, XP002106860 ISSN: 8756-7938
- PATENT ABSTRACTS OF JAPAN vol. 1998, no. 04, 31. März 1998 (1998-03-31) & JP 09 322755 A (RIKAGAKU KENKYUSHO & SAINIKUSU K.K.), 16. Dezember 1997 (1997-12-16) & DATABASE WPI Section Ch, Week 199810 Derwent Publications Ltd., London, GB; Class B04, AN 1998-103992 & JP 09 322755 A (RIKAGAKU KENKYUSHO & SAINIKUSU K.K.), 16. Dezember 1997 (1997-12-16)

## Beschreibung

Die Erfindung betrifft ein Verfahren sowie eine Vorrichtung zur Durchführung biochemischer Reaktionen, insbesondere für die Polypeptid-Biosynthese bzw. zur gekoppelten in vitro-Transkription und Translation von Proteinen in einem zellfreien System mit Hilfe einer "Multi-channel"-Dialyse-Vorrichtung, wodurch die Synthese unterschiedlicher Proteine nebeneinander in ausreichenden Ausbeuten unter einfachen und reproduzierbaren Bedingungen möglich ist.

Das Prinzip der zellfreien in vitro-Proteinbiosynthese unter Einsatz von Dialysemembranen ist seit mehreren Jahren bekannt und besteht im wesentlichen darin, daß zwei getrennte Kammern (eine für die Reaktionsmischung, eine für die Versorgungslösung) über eine Membran mit geeigneter Porengröße in Verbindung stehen. In der Reaktionskammer erfolgt die Proteinsynthese. In der Versorgungskammer ist eine Lösung mit sämtlichen für die Transkription bzw. Translation benötigten Reaktionskomponenten, die während der laufenden Proteinbiosynthese in der Reaktionsmischung verbraucht weiden, enthalten. Bedingt durch die Tatsache, daß beide Kammern über eine semipermeablen Membran in Verbindung stehen, können verbrauchte Reaktionskomponenten in der Reaktionskammer, gegebenenfalls mittels einer entsprechenden Pumpeinrichtung, fortlaufend durch neue Komponenten aus der Versorgungskammer ersetzt werden, wodurch die Synthese über einen deutlich längeren Zeitraum im Vergleich zum statischen System, d.h. einer entsprechenden, in einem nicht unterteilten Reaktionsgefäß stattfindenden Reaktion, aufrechterhalten bleibt. Entsprechende Verfahren für die zellfreie in vitro-Biosynthese nach dem "continous-flow" bzw. "continous exchange"-Prinzip sind beispielsweise in US 5.478.730, EP 0 593 757 und von Spirin et al. in Science vol. 242, 1988, S. 1162-1164 beschrieben.

Das gemäß der Patentschrift US 5.478.730 verwendete Synthesesystem enthält beispielsweise eine Quelle von DNA bzw. mRNA, die das Polypeptid codiert. Weiter sind in dem zellfreien Synthesesystem im wesentlichen Ribosomen, tRNA, Aminosäuren, ATP, GTP, UTP und CTP enthalten. Die Transkription der DNA bzw. die Translation der mRNA mit Hilfe der tRNA führt zur Produktion des jeweiligen Polypeptids, wobei zugleich Nebenprodukte und Abfallstoffe mit niedrigerem Molekulargewicht entstehen. Diese können durch eine semipermeable Membran, die den Raum, in dem das Synthesesystem enthalten ist, von einem Versorgungsraum trennt, in den Versorgungsraum übertreten. Der Versorgungsraum enthält eine als Versorgungsmedium wirkende Flüssigkeit, in der insbesondere ATP, GTP und Aminosäuren enthalten sind. Diese Komponenten werden durch die semipermeable Membran dem Synthesesystem zugeführt, um den Verbrauch während der Biosynthesereaktion zu ersetzen. Der Durchtritt durch die semipermeable Membran ist möglich, weil ihr Molekulargewicht unterhalb von deren Durchlaßgrenze liegt. Zugleich gelangen Produkte der biochemischen Reaktion und andere Substanzen, deren Molekulargewicht unterhalb der Durchlaßgrenze der Barriere liegt, aus dem Reaktionsraum in den Versorgungsraum. Die semipermeable Membran ist gemäß der US Patentschrift 5.478.730 beispielsweise eine Ultrafiltrationsmembran in Form von Membran-Hohlfasern.

Die US Patentschrift 5.478.730 enthält umfangreiche weitere Erläuterungen über geeignete Zusammensetzungen des Synthesesystems und der Versorgungsflüssigkeit. Insoweit bezieht sich die vorliegende Erfindung auf den vorbekannten Stand der Technik, wie er insbesondere dieser US Patentschrift und den darin zitierten Literaturstellen zu entnehmen ist. Deren Inhalt wird durch Bezugnahme zum Inhalt der vorliegenden Anmeldung gemacht.

Das europäische Patent 0 593 757 beschreibt die Anwendung der gekoppelten Transkription und Translation für entsprechende in vitro-Biosyntheseverfahren, wobei im wesentlichen zusätzlich eine RNA-Polymerase der Reaktionsmischung zugesetzt wurde.

Darüber hinaus sind diverse Dialyse-Vorrichtungen bzw. -Membranen bekannt. Man unterscheidet dabei im wesentlichen sogenannte "single tube" und "Multi-channel"-Ausführungen. Die beschriebenen bzw. kommerziell erhältlichen und für die in vitro-Proteinbiosynthese geeigneten Dialysematerialien beruhen ausschließlich auf dem "single tube"-Prinzip (z.B. EP 99 91 01 418.4; Promega Notes 1997). Ein hoher Durchsatz bzw. die Synthese unterschiedlicher Proteine nebeneinander, darüber hinaus ohne aufwendige Aufreinigungsmaßnahmen, oder anderen Maßnahmen, wie z.B. für die Denaturierung von als "inclusion bodies" anfallenden Proteinen, ist mittels dieser Dialyse-Hilfsmittel nicht möglich. Ferner sind die Dialyse-Vorrichtungen nach dem "single-tube"-Prinzip mit dem Nachteil verbunden, daß sie technisch und zeitlich aufwendig zu handhaben sind.

Ferner werden Mikrotiterplatten mit Vertiefungen, welche am unteren Ende mit einer porösen Membran versehen sind, kommerziell angeboten. Sogenannte "Multi-channel"-Ausführungen sind ausschließlich für Zellkulturanwendungen bzw. für Filtrations- oder Umpufferungsvorgänge geeignet (z.B. Ultrafiltrationsmembranen von Milipore; Slide-A-Lyzer® MINI Dialysis Unit von Pierce). Ein Austausch zwischen Flüssigkeiten unterschiedlicher Konzentrationen, wie sie bei der in vitro-Transkription bzw. in vitro-Translation verwendet werden, ist mit diesen "Multi-channel"-Vorrichtungen jedoch nicht möglich, weshalb diese für in vitro-Proteinsynthese-Anwendungen, beispielsweise mit kontinuierlicher Zufuhr bzw. Entnahme von Komponenten nicht geeignet sind.

Ferner sind Lösungs- bzw. Reagenzkombinationen bekannt, die für die Durchführung von in vitro-Proteinsynthesen geeignet sind. Gemäß US 5.593.856 sind die erforderlichen Komponenten vor dem Start der Reaktion insbesondere in zwei Stammlösungen enthalten, einem sogenannten Reaktionsmedium und einem zellfreien Extrakt. Die Nukleotidtriphosphate und die energiereiche Verbindung (z.B. Acetylphosphat) befinden sich in ein und derselben Lösung. Ferner sind Reaktions- bzw. Versorgungslösungen beschrieben, bei denen sämtliche für die in vitro-Proteinsynthese erforderlichen Komponenten bevorzugt in einem Reaktionsgefäß vereinigt vorliegen (WO 99/50436). Ein Hinweis dahingehend, dass entsprechende Lösungs- bzw. Reagenzkombinationen zur Lagerung geeignet sind, ist dem Stand der Technik nicht zu entnehmen.

Der Erfindung liegt somit die Aufgabe zugrunde, Lösungs- bzw. Reagenkombinationen zur Verfügung zu stellen, die für die Durchführung von in vitro-Proteinsynthesen und zugleich zur Lagerung geeignet sind.

Die Aufgabe wird gelöst durch einen Reaktionskit zur Durchführung von in vitro-Proteinsynthesen bzw. zur gekoppelten in vitro-Transkription und Translation von Proteinen im zellfreien System mit einer entsprechenden Vorrichtung. Der Kit besteht im wesentlichen aus einer Versorgungslösung und einer Lösung für den bzw. die Reaktionsansätze. Die Lösungen können in flüssiger Form sowie in gefriergetrocknetem Zustand vorliegen, wobei die von den Komponenten der Versorgungslösung sich unterscheidenden Komponenten jeweils in separaten Gefäßen vorliegen. Die Versorgungslösung beinhaltet im wesentlichen eine zwischen pH 7 und 8 puffernde Substanz, ca. 150 bis 400 mM Kaliumionen, ca. 10 bis 50 mM Magnesiumionen, Nukleotidtriphosphate (ATP, CTP, GTP und UTP), ca. 20 unterschiedliche Aminosäuren und eine Sulfidgruppen reduzierende Substanz. Darüber hinaus können der Versorgungslösung weitere Hilfsstoffe wie Stabilisatoren oder Inhibitoren zur Vermeidung nicht erwünschter Reaktionen zugesetzt sein. Die Lösung für die Reaktionsansätze entspricht der Lösung der vorgenannten Versorgungslösung und umfaßt darüber hinaus ein zellfreies Lysat, d.h. eine prokaryotische oder eukaryotische Ribosomenfraktion, tRNA und eine RNA-Polymerase, deren Ursprung von der der Ribosomenfraktion unterschiedlich ist. Erfindungsgemäß besonders bevorzugt ist, wenn die von den Komponenten der Versorgungslösung sich unterscheidenden Komponenten der Lösung für die Reaktionsansätze erst kurz vor Durchführung der Reaktion beigemengt werden, d.h. jeweils in separaten Gefäßen vorliegen. Entsprechendes gilt für sowohl der Versorgungslösung als auch der Reaktionslösung zuzusetzende energiereiche Verbindungen wie z.B. Acetylphosphat. Dadurch wird die Lagerfähigkeit und die Adaptionsfähigkeit des Reaktionskits weiter verbessert.

Ein weiterer Gegenstand der Erfindung ist eine Vorrichtung zur Durchführung biochemischer Reaktionen, insbesondere für die zellfreie Polypeptid-Biosynthese und/oder für die Herstellung biologisch aktiver und nativ struktrierter Proteine, bestehend aus einem äußeren Gehäuse, das ein inneres Gehäuse mit eingearbeiteten Vertiefungen und eine Versorgungskammer umschließt, wobei die Vertiefungen des inneren Gehäuses während der biochemischen Reaktion jeweils ein produzierendes System enthalten, die Versorgungskammer eine Versorgungsflüssigkeit enthält und die Vertiefungen des inneren Gehäuses und die Versorgungskammer durch eine semipermeable Membran getrennt sind, dadurch gekennzeichnet, daß das innere Gehäuse mindestens zwei Vertiefungen aufweist, deren untere Enden mit der semipermeablen Membran abgeschlossen sind und deren obere Enden aus der in der Versorgungskammer enthaltenen Versorgungsflüssigkeit herausragen, und mit Mitteln zur Bewegung und Temperierung der produzierenden Systeme sowie der Versorgungsflüssigkeit verbunden ist.

Als äußere Gehäuse kommen erfindungsgemäß beispielsweise schalen- oder zylinderförmige Gefäße in Betracht, die zur Flüssigkeitsaufnahme befähigt sind und in denen ein geometrisch kleineres, inneres Gehäuse einsetzbar und gegebenenfalls fixierbar ist. Das innere Gehäuse kann, entsprechend dem äußeren Gehäuse, größen- und formgemäß beliebige äußere Ausmaße haben. Beispielsweise sind runde, rechteckige oder quadratische Anordnungen für das äußere Gehäuse mit Volumina von ca. 10 ml bis mehrere Liter geeignet. Vorteilhaft ist, wenn das innere Gehäuse möglichst weitgehend an die Geometrie des äußeren Gehäuses angepaßt ist. Ferner kann es sich bei dem inneren Gehäuse um ein Mikrotiterplatten (MTP)-Format handeln. Dadurch ist eine einfache Automatisierung bzw. Beschleunigung der einzelnen Arbeitsschritte möglich.

Das innere Gehäuse weist eine oder mehrere Vertiefungen auf, bevorzugt ist eine Anzahl zwei oder mehr, z. B. sechs, acht, zwölf, 24, 48, 64, 96, 384 etc. Es sind jedoch prinzipiell auch innere Gehäuse, beispielsweise in Form von Blöcken oder Mikrotiterplatten, die mehrere hundert bis 1000 oder mehr Vertiefungen für die erfindungsgemäße Vorrichtung aufweisen, einsetzbar. Die Vertiefungen sind in der Regel aus einem inertem Material, wie beispielsweise Polyethylen oder Polypropylen, gefertigt und können für Volumina von ca. 50 µl bis zu mehreren Millilitern, d.h. in der Größenordnung von 10 ml ausgerichtet sein. Vorteilhaft sind konisch zulaufende Vertiefungen, die am unteren Ende mit einer semipermeablen Membran, wie z. B. einer Dialysemembran mit einer Porengröße von 3 bis 100 Kilo Dalton, verschlossen sind. Prinzipiell können erfindungsgemäß sämtliche gängigen Dialyse- bzw. Ultrafiltrationsmembranen mit entsprechender Porengröße eingesetzt werden. Besonders geeignet haben sich Dialysemembranen mit einer Porengröße von ca. 10 bis 14 Kilo Dalton erwiesen. Dadurch können insbesondere störende niedermolekulare, inhibitorische Substanzen, die bei der in vitro-Biosynthese entstehen, abgetrennt werden. Zur Abdichtung der oberen Öffnungen der Vertiefungen, d.h. des aus der in der Versorgungskammer enthaltenen Versorgungsflüssigkeit herausragenden Teils der Vertiefungen des inneren Gehäuses, kann entweder jede Vertiefung für sich oder auch alle Vertiefungen gemeinsam mit einer Verschlußkappe oder Folie versehen sein. Alternativ kann auch ein Verschlußdeckel zur Abdichtung des äußeren Gehäuses insgesamt angebracht sein.

Eine weitere bevorzugte Ausführungsform des inneren Gehäuses der erfindungsgemäßen Vorrichtung besteht aus einer Schicht von Blöcken mit einer Vielzahl von Bohrungen. Über einem ersten Block befindet sich ein weiterer, flacher Block mit der gleichen Bohrungsgeometrie. Zwischen beide Blöcke wird eine Filter- bzw. semipermeable Membran eingebracht. Der zweite flachere Block ist zum Auffangen von Nebenprodukten bzw. Abfallstoffen geeignet, die somit nicht bzw. lediglich in sehr geringem Umfang in den Versorgungsraum gelangen. Im Ergebnis erhält man ein inneres Gehäuse mit Vertiefungen, welche jeweils durch eine Membran in Reaktionsraum und eine sogenannte zweite Versorgungs- bzw. Dialysekammer unterteilt sind. Durch beidseitigen Abschluß des inneren Gehäuses durch Dialysemembranen wird einerseits die für den Austausch zur Verfügung stehende Fläche verdoppelt und andererseits kommt es dadurch kaum zu Volumenänderungen in den Proben. Besonders vorteilhaft bei dieser Ausführungsform ist, daß sich in der die in vitro-Proteinsynthese unterhaltenden Versorgungsflüssigkeit nicht verwertbare Komponenten weit weniger bzw. zeitlich verzögert anreichern, wodurch die Effektivität der Proteinsynthese weiter erhöht wird.

Darüber hinaus hat sich als vorteilhaft erwiesen, wenn die Wände der einzelnen Vertiefungen ("Wells") des inneren Gehäuses mit die in vitro synthetisierten Proteine bzw. Peptide spezifisch bindenden Komponenten beschichtet sind. Entsprechende Komponenten sind insbesondere solche, die zur Reinigung von "tag"-enthaltenden Proteinen geeignet sind. Das in den Wells in vitro synthetisierte Protein (tag enthaltend) kann so an die beschichtete Mikrotiterplatte gebunden und anschließend direkt, gegebenenfalls nach Waschen mit geeigneten Puffern, gereinigt werden bzw. durch geeignete Reagenzien in sauberer Form eluiert werden. Als sogenanntes Protein-tag ist beispielsweise Strep-Tag II (8AS-Sequenz, s. DE 42 37 113), das entweder N- oder C-terminal an das in vitro-synthetisierte Protein gebunden sein kann, anzuführen. Als Beschichtungssubstanzen können beispielsweise Streptactin, Streptavidin oder Avidin eingesetzt werden. Verfahren zur Beschichtung entsprechender Oberflächen sind dem Fachmann prinzipiell bekannt. Alternativ ist auch möglich, die synthetisierten, tag-tragenden Proteine mittels entsprechend beschichteter Glas- bzw. Magnetpartikel aus der Reaktionsmischung der einzelnen Vertiefungen zu separieren.

Ferner ist die erfindungsgemäße Vorrichtung mit einer Rühr- oder Schüttelvorrichtung ausgestattet, um eine ausreichende Bewegung bzw. Diffusion der Reaktionslösung(en) und der Versorgungslösung zu garantieren. Als vorteilhaft hat sich erwiesen, wenn in der Versorgungskammer und gegebenenfalls in jeder einzelnen Vertiefung, in der eine Reaktion abläuft, ein Rührelement in Form eines Magnetrührers eingebracht ist. Dadurch wird eine simultane Durchmischung des produzierenden Systems bzw. der Mischungen in den einzelnen Vertiefungen und der umliegenden Versorgungslösung gewährleistet. Zur Gewährleistung einer konstanten Temperatur während der biochemischen Reaktion - welche in der Regel zwischen 20° und 37°C beträgt - wird die gesamte Vorrichtung am einfachsten vollständig in einen verschließbaren Inkubator eingebracht oder unter einer temperierbaren Inkubationshaube gehalten. Darüber hinaus sind erfindungsgemäß kombinierte Schüttel- bzw. Rühr- und Thermostatisiergerät einsetzbar. Die Durchmischung der Reaktionsmischung(en), als auch der Versorgungslösung kann somit simultan mit Schüttel- bzw. Rührbewegungen geeigneter Frequenz sowie über einen längeren Zeitraum bei konstanter Temperatur erfolgen.

In der Regel ist ausreichend, die Reaktionsmischung(en) über einen Zeitraum von ca. 20 Stunden zu inkubieren, um das bzw. die gewünschten Proteine bzw. Peptide in ausreichenden Ausbeuten zu erhalten. Abhängig von dem zu synthetisierenden Protein bzw. der Optimierung der einzelnen Verfahrensparameter bzw. der genauen Zusammensetzung der Versorgungslösung können bereits nach ca. 6 Stunden ca. 25 bis 50 µg Protein /250 µl Reaktionslösung in einer Vertiefung (Well), was einer Konzentration von 100 bis 200 µg/ml entspricht, erzielt werden. Durch verlängerte Inkubationszeiten können darüber hinaus entsprechend bessere Ausbeuten erreicht werden, beispielsweise wurden für GFP (Green Fluorescent Protein) Konzentrationen bis zu 500 µg/ml erhalten.

Eine weitere bevorzugte Ausführungsform der Erfindung ist, wenn abhängig von der Anzahl bzw. dem Gesamtvolumen der Vertiefungen des inneren Gehäuses das Volumen der Versorgungslösung bestimmt wird. Als Richtlinie gilt erfindungsgemäß, daß das Volumen der Versorgungslösung gleich der Summe aus der Anzahl der Vertiefungen und dem Volumen pro Vertiefung multipliziert mit dem Faktor 10 entspricht.

Die Zusammensetzung der Versorgungslösung entspricht im wesentlichen entsprechenden Lösungen für die zellfreie in vitro-Biosynthese des Standes der Technik. Dem Fachmann ist darüber hinaus geläufig, daß Versorgungslösungen für die zellfreie Proteinsynthese bestimmten üblichen Optimierungsmaßnahmen unterliegen, insbesondere je nach Art und Beschaffenheit der verwendeten Ribosomenfraktion, d.h. ob beispielsweise ein eukaryontisches oder prokaryontisches System als Grundlage für die zellfreie in vitro-Biosynthese verwendet wird. Als vorteilhaft hat sich darüber hinaus erwiesen, wenn die Versorgungslösung ein Sulfidgruppen reduzierendes Mittel sowie - im Falle eines Lysats auf E. coli-Basis - einen Inhibitor für E. coli-Polymerasen, und gegebenenfalls eine oder mehrere geeignete bakterizide Substanzen enthält.

Eine erfindungsgemäß besonders vorteilhafte Versorgungslösung für eine gekoppelte Transkriptions-/Translationsreaktion umfaßt in einem geeigneten Puffersystem wie z.B. Hepes, ca. 150 bis 400 mM Kaliumionen, ca. 10 bis 50 mM Magnesiumionen, ausreichende Mengen der vier Nukleotidtriphosphate (ATP, CTP, GTP und UTP) sowie sämtlicher natürlich vorkommender Aminosäuren, ca. 20 bis 80 mM Acetylphosphat, Dithiothreitol als Sulfidgruppen reduzierendes Reagenz sowie gegebenenfalls EDTA, Glyzerin, eine oder mehrere bakterizide Substanzen wie z.B. Rifampicin oder Natriumazit und bevorzugt - im Fall einer Ribosomenfraktion auf E. coli-Basis - einen RNA-Polymerasen-Inhibitor wie z.B. Rifampicin zur Deaktivierung von E. coli-Polymerasen. Ein typischer Reaktionsansatz für eine Transkriptions-/Translationsreaktion enthält entsprechende Komponenten in vergleichbaren Mengen wie die Versorgungslösung. Darüber hinaus beinhaltet ein erfindungsgemäßer Reaktionsansatz die jeweilige eukaryotische oder prokaryotische Ribosomenfraktion wie z.B. ein E. coli-Lysat, die für das gewünschte Protein kodierende DNA in Form eines Plasmids, ca. 1 bis 10 U/µl einer RNA-Polymerase, ca. 200 bis 800 µg/ml einer tRNA sowie gegebenenfalls weitere Hilfsstoffe, wie z.B. RNase-Inhibitoren.

Ein weiterer Gegenstand der Erfindung ist ein Verfahren zur Durchführung einer und insbesondere mehrerer parallel laufender biochemischer Reaktionen mittels der erfindungsgemäßen Vorrichtung, wobei während der biochemischen Reaktion die Versorgungsflüssigkeit in der Versorgungskammer nicht einem äußerlich auferlegtem Druck unterliegt, so daß der molekulare Austausch zwischen der Versorgungskammer und den Lösungen der einzelnen Vertiefungen des inneren Gehäuses im wesentlichen auf Diffusion basiert.

Das erfindungsgemäße Verfahren bzw. die hierfür geeignete Vorrichtung eignet sich insbesondere für automatisierte Anwendungen mit hohem Synthesedurchsatz.

Durch die nachfolgenden Beispiele wird die Erfindung weiter erläutert.

### Beispiel 1:

Das erfindungsgemäße Verfahren zur zellfreien Proteinbiosynthese wird mit einem E. coli Lysat an zwei Modellproteinen (CAT und GFP) in folgendem näher erläutert:
Die zellfreie Proteinbiosynthese wird in Form einer gekoppelten Transkription und Translation durchgeführt, bei der die zu transkribierende mRNA auf einem Plasmid kodiert ist, dessen Gensequenz einen Promotor für eine virale RNA-Polymerase (z. B. SP6-, T3- oder T7-RNA-Polymerase) enthält.
Die in vitro transkribierte mRNA wird mit Hilfe des im gekoppelten Systems befindlichen E. coli Lysats in das entsprechende Protein translatiert.

### A) Reaktionskomponenten:

| | | |
|---|---|---|
| *Plasmide:* | pM-GFP oder plVEX-GFP enthalten die Sequenz für das Green Flourescent Protein aus Aequorea victoria in Form einer Mutante GFPryde3 (27 kiloDalton) (Nature Biotechnology, 1996, 14, p.315-319); die kodierende Region der GFPcycle3 Mutante wurde in pTU58 anstelle der Wild-Typ GFP-Sequenz kloniert (Science, 1994,263,802). | |
| | pHM-CAT enthält die Sequenz für das Chloramphenicol-Acetyltransferase-Protein (22,5 kiloDalton). | |
| | Konstruktion: | Ein Insert (Ncol - BamHI) aus pCAT3 (Promega) wurde in pHM19 insertiert (FU Berlin, Insitut f. Biochemie, Dr. Stiege). |
| *E.coli S30 Lysat:* | Das Lysat wurde mit einem E. coli A19-Stamm nach einem modifizierten Verfahren nach Zubay (Annu. Rev. Genet. 7,267, 1973) präpariert. | |
| | Lysatpuffer: | 100 mM Hepes-KOH pH 7,6/30°C, 14 mM Magnesium-acetat, 60 mM Kalium-acetat, 0,5 mM Dithiothreito |

### Zusammensetzung der Reaktions- und Versorgungslösung:

### Transkriptions-/Translations-Reaktionsansatz:

185 mM Kalium-acetat, 15mM Magnesium-acetat, 4% Glyzerin, 2,06 mM ATP, 1,02 mM CTP, 1,64 mM GTP, 1,02 mM UTP, 257 µM pro Aminosäure (insgesamt 20), 10,8 µg/ml Folinsäure, 1,03 mM EDTA, 100 mM Hepes-KOH pH 7,6/30°C, 1 µg/ml Rifampicin, 0,03% Natriumazit, 40 mM Acetylphosphat, 480 µg/ml t RNA aus E.coli MRE600, 2mM Dithiothreitol, 10mM MESNA (Mercaptoethanesulfonic acid), 70 mM Kaliumhydroxid, 0,1 u/µl RNaseInhibitor, 15 µg/ml Plasmid, 220 µl/ml E.coli A19-Lysat, 2 U/µl T7 RNA Polymerase.

### Versorgungslösung:

185 mM Kalium-acetat, 15 mM Magnesium-acetat, 4% Glyzerin, 2,06 mM ATP, 1,02 mM CTP, 1,64 mM GTP, 1,02 mM UTP, 257 µM pro Aminosäure (insgesamt 20), 10,8 µg/ml Folinsäure, 1,03 mM EDTA, 100 mM Hepes-KOH pH 7,6/30°C,1 µg/ml Rifampicin, 0,03% Natriumazit, 40 mM Acetylphosphat, 2 mM Dithiothreitol, 10 mM MESNA (Mercaptoethanesulfonic acid), 70 mM Kaliumhydroxid, Lysatpuffer wie oben beschrieben mit 220 µl/ml.

### B) Vorrichtung:

In dem hier beschriebenem Beispiel wurde ein Multi-channel-Dialyzer in Form einer Mikrotiterplatte eingesetzt. Die Böden der einzelnen Vertiefungen der Mikrotiterplatte (Wells) wurden mit einer Dialysemembran mit einem cut off-Volumen von 10 Kilo Dalton ausgestattet. Das maximale Reaktionsvolumen pro Well betrug 200 µl. Das Gehäuse für die Versorgungslösung hatte eine Volumenkapazität von 200 ml.

### C) Bewegung, Abdeckung:

Schüttelgerät: Flow Laboratories, Typ: Titertek®, mit der Schüttelfrequenz Stufe 4,5
Inkubationshaube: Edmund Bühler, Typ: TH25, mit der Temperatur 30°C

### D) Durchführung:

Es wurden getrennte Reaktionsansätze gemäß oben angegebener Zusammensetzung pipettiert:
1) Für die Expression von GFP mit pM-GFP bzw. mit pIVEX-GFP
2) Für die Expression von CAT mit pHM-CAT

Für jeden Ansatz wurde ein Volumen von 8,4 ml angesetzt und davon jeweils 200 µl in insgesamt 40 Wells pipettiert (0,4 ml als Überdosierung), das heißt 40 Wells pro Ansatz und Protein.

Für die Versorgungskammer wurden 200 ml Versorgungslösung gemäß oben angegebener Zusammensetzung pipettiert. Das heißt, es wurde eine gemeinsame Versorgungslösung für beide Reaktionsansätze verwendet.

Nach Abdichten der Mikrotiterplatte mit einer Klebefolie wurde die Vorrichtung auf das Schüttelgerät fixiert, welches sich unter der Inkubationshaube befand.

Nach Einstellen der Schüttelfrequenz und der Inkubationstemperatur von 30°C erfolgte eine 20 stündige Inkubation der Reaktionsansätze.

### E) Auswertung:

### 1) Expression von GFP:

Die Messung der Proben erfolgte mit einem Spektral-Fluorimeter der Fa. Kontron, Typ: TEGIMENTA, SFM25.
Die Anregung (Excitation) erfolgt bei einer Wellenlänge von 395 nm.
Die Emissionsrate bei 580 bis 430 nm.
Das Emissionsmaximum liegt bei 510 nm.

Als Standard wurde das rGFP (rekombinantes GFP) von Roche Diagnostics, Katalog-Nummer 1814524 verwendet.

Die Proben wurden mit dem Lysatpuffer 1 : 200, der Standard auf 1 µg/ml und 2 µg/ml verdünnt.

### Tabelle 1:

10 Werte der erhaltenen 40 (die alle im selben Bereich liegen) sind als representative Auswahl gezeigt.

### Abbildung 1:

Graphische Auswertung der in Tabelle 1 enthalenen Werte in µg GFP/ml Reaktionslösung in den exemplarisch ausgewählten Wells.

**Tabelle 1:**

| Well-Nummer | 1 | 2 | 3 | 4 | 5 | 6 | 7 | 8 | 9 | 10 |
|---|---|---|---|---|---|---|---|---|---|---|
| µg GFP/ml Reakt.vol. | 366 | 365 | 362 | 356 | 369 | 371 | 365 | 367 | 361 | 365 |

### 2) Expression von CAT:

Die Messung der Proben erfolgte über HPLC-Analyse mit dem Geräte-Typ:.LKB 2150.

Als Standard wurde ein CAT-Enzym von Roche Diagnostics, Katalog-Nummer 1485156 verwendet.

### Tabelle 2:

10 Werte der erhaltenen 40 (die alle im selben Bereich liegen) sind als representative Auswahl gezeigt.

### Abbildung 2:

Graphische Auswertung der in Tabelle 2 enthaltenen Werte in µg CAT/ml Reaktionslösung in den exemplarisch ausgewählten Wells.

**Tabelle 2:**

| Well-Nummer | 1 | 2 | 3 | 4 | 5 | 6 | 7 | 8 | 9 | 10 |
|---|---|---|---|---|---|---|---|---|---|---|
| µg CAT/ml Reaktionslsg. | 233 | 230 | 233 | 235 | 228 | 231 | 227 | 228 | 229 | 232 |

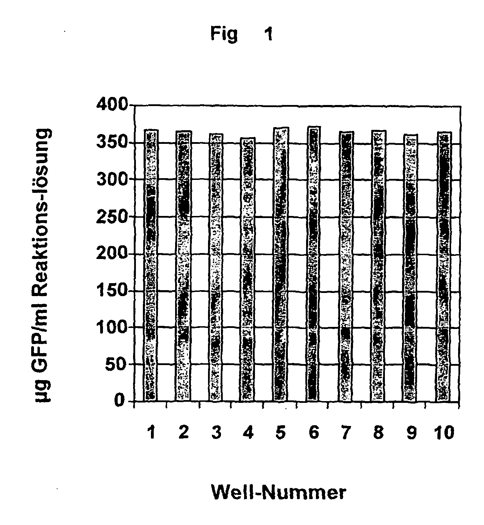

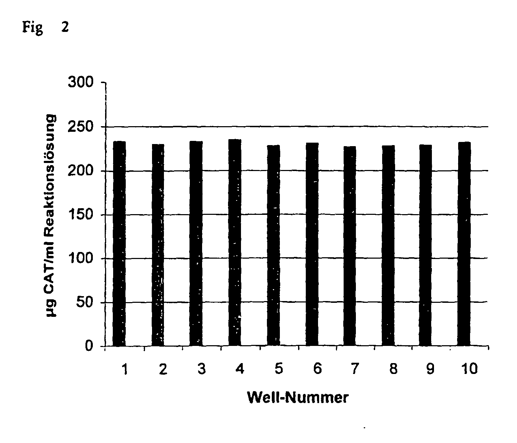

## Patentansprüche

1. Kit zur Durchführung von in vitro-Proteinsynthesen bestehend aus folgenden Komponenten
1) einer Versorgungslösung, welche eine zwischen pH 7 und 8 puffernde Substanz, 150 bis 400 mM Kaliumionen, 10 bis 50 mM Magnesiumionen, Nukleotidtriphosphate, Aminosäuren und eine Sulfidgruppen reduzierende Substanz enthält,
2) einer energiereichen Verbindung,
3) einer tRNA-Fraktion,
4) einer RNA-Polymerase und
5) einem zellfreien Lysat, **dadurch gekennzeichnet, dass** die von den Komponenten der Versorgungslösung sich unterscheidenden Komponenten jeweils in separaten Gefäßen vorliegen.

2. Kit gemäß Anspruch 1, **dadurch gekennzeichnet, dass** der Ursprung der RNA-Polymerase von dem der Ribosomenfraktion des zellfreien Lysats unterschiedlich ist.

## Claims

1. Kit for carrying out in vitro protein syntheses composed of the following components
1) a supply solution which contains a substance buffering between pH 7 and 8, 150 to 400 mM potassium ions, 10 to 50 mM magnesium ions, nucleotide triphosphates, amino acids and a substance reducing sulphide groups,
2) an energy-rich compound,
3) a tRNA fraction,
4) an RNA polymerase and
5) a cell-free lysate, **characterized in that** the components which differ from the supply solution are each present in separate vessels.

2. Kit according to claim 1, **characterized in that** the origin of the RNA polymerase is different from that of the ribosomal fraction of the cell-free lysate.

## Revendications

1. Kit destiné à la synthèse in vitro de protéines, contenant les composants suivants :
1) une solution d'alimentation qui contient une substance ayant un effet tampon entre pH 7 et 8, 150 à 400 mM d'ions potassium, 10 à 50 mM d'ions magnésium, des nucléotide-triphosphates, des acides aminés et une substance réduisant les groupes sulfure,
2) un composé riche en énergie,
3) une fraction d'ARNt,
4) une ARN polymérase et
5) un lysat exempt de cellules, **caractérisé en ce que** les composants autres que les composants de la solution d'alimentation sont disposés chacun dans un récipient séparé.

2. Kit selon la revendication 1, **caractérisé en ce que** l'origine de l'ARN polymérase est différente de la fraction ribosomique du lysat exempt de cellules.
